# NEW EUROPEAN PATENT SPECIFICATION

(11) **EP 2 552 539 B2**
(45) Date of publication and mention of the opposition decision: **06.03.2019**
(45) Mention of the grant of the patent: 26.08.2015
(21) Application number: 10712906.6
(22) Date of filing: 01.04.2010
(51) Int. Cl.: A61N 1/36, A61F 9/08

(54) **RETINAL IMPLANT AND VISUAL PROSTHESIS INCORPORATING SUCH AN IMPLANT**
RETINAIMPLANTAT UND SEHPROTHESE MIT EINEM DERARTIGEN IMPLANTAT
IMPLANT RÉTINIEN ET PROTHÈSE VISUELLE INCORPORANT UN TEL IMPLANT

(43) Date of publication of application: 06.02.2013
(73) Proprietor: Pixium Vision SA, 75012 Paris (FR)
(72) Inventor: KLAVER, Tom, 51065 Köln (DE); DAPPER, Marcus, 53227 Bonn (DE); TIEDTKE, Hans-Jürgen, 53227 Bonn (DE)
(74) Representative: Graf von Stosch, Andreas
(86) International application number: PCT/EP2010/002112
(87) International publication number: WO 2011/120540

(56) References cited:
- WO-A2-2007/006376
- US-A- 5 865 839
- US-A1- 2002 087 202
- US-A1- 2004 030 383
- US-A1- 2004 088 026
- US-A1- 2005 090 875
- US-A1- 2006 142 818
- US-A1- 2007 142 877
- US-A1- 2007 191 909
- US-A1- 2008 086 206
- US-B1- 6 298 270
- US-B2- 6 921 413

## Description

### Technical Field

The present invention relates to a medical implant device, and more particularly to a retinal or ocular implant for a visual prosthesis for use in artificially generating vision in a patient suffering from partial or total vision loss.

### Background Art

Since the 1990s various research groups have been working on the development of a visual prosthesis that would allow at least partial restoration of sight to individuals suffering from certain forms of vision loss or blindness, most notably from the effects of retinitis pigmentosa and/or age-related macular degeneration. The majority of the prostheses or systems for generating artificial vision that have been developed to date comprise an ocular implant designed for electrically stimulating the functional nerves of the retina. One example of such a system is described in the International Patent Application Publication No. WO 2007/006376 A2. That system includes a camera for capturing an image, which is then processed and transmitted as electric signals to an electrode array provided on the implant to electrically stimulate the retinal nerve cells. The system employs a wireless power supply via induction, and employs telemetry for data transfer, either as RF signals or as infrared light. A retinal prosthesis according to the preamble part of claim 1 and a system according to the preamble part of claim 9 are known from US 2004/088026 A1, US 2004/030383 A1, US 2007/142877 A1, and US 2006/142818 A1.

WO 2010/105728 A2 (prior art under Art. 54(3) EPC) describes a visual prosthesis for optically stimulating nerve cells of the retina by infrared radiation.

Because the environment within the eye is aqueous, problems arise in association with the penetration of solutes or moisture into the implants and/or electrochemical reactions involving the electrodes. As the electrodes project from the implant for direct physical contact with the nerve tissue of the retina, they are naturally exposed to the aqueous environment. The electrodes, which are typically made of platinum-coated gold, tend to dissolve in this environment as a result of the application of a direct current to stimulate the nerve tissue. In the interests of a highly localized or specific stimulation, and thus good vision resolution, the electrodes are desirably made as small as possible. But the impact of electrochemical degradation increases with diminishing size and this, in turn, limits size reduction. Furthermore, the locations where the electrodes physically emerge from the sealed implant present potential sites for the ingress of moisture into the electronics or circuitry of the implant. For example, the conductive traces that electrically connect each of the electrodes are extremely fine or thin so that any ingress of moisture may also expose those traces to electrochemical degradation. Apart from electrochemical processes, the de-lamination of the sheath or coating that hermetically seals the implant can also be highly damaging.

In addition to electrode size, another factor which may affect the resolution of the artificially generated vision is electrical interference between electrodes, also known as 'cross-talking'. The electrical current directed into the tissue from each electrode tends to spread through the tissue around that electrode. Thus, at the threshold current necessary to evoke a depolarisation of the nerve cells, there is a limit to the proximity of electrodes to each other. If the electrodes are too close together, the stimulus field from one electrode can overlap and interfere with a region of nerve tissue served by another electrode, with unwanted stimulation as a result. Accordingly, not only should the electrodes have at least a certain size, they should also be spaced at a sufficient distance from each other to prevent 'cross-talking'.

The greater the proximity of the electrodes to the nerve cells to be stimulated, the smaller the currents that are required for stimulation. Although the 'cross-talking' effect can be reduced by using smaller currents, thereby enabling the electrodes to be spaced closer together, the electrodes must then penetrate into the retina tissue to be very close to the nerve cells to be stimulated. A significant drawback of this arrangement is that, if the implant needs to be replaced for some reason, extracting the electrodes will almost certainly cause serious damage to the retinal tissue.

Thus, it is an object of the present invention to provide a medical implant device, and in particular an implant for a visual prosthesis, with which one or more of the above the drawbacks or limitations may be substantially overcome.

### Summay of the Invention

The present invention provides a retinal implant or prosthesis as recited in claim 1. Preferred features of the invention are recited in the dependent claims.

According to one aspect, the present invention provides a visual prosthesis or a system for generating artificial vision in a subject, comprising:
image capture means to capture an image from a surrounding environment;
image processing means to process the image and convert the image into an image signal; and
a retinal prosthesis or stimulation device according to the invention configured to be implanted within an, eye of a patient and positioned in, on or adjacent the retina, the retinal prosthesis or stimulation device comprising: a support layer or substrate and a plurality of light sources arranged on the substrate to stimulate nerve cells of the retina,
wherein the plurality of light sources are configured to emit infrared radiation in response to stimulation signals derived from the image signal.

It will be understood that the references to "light sources" in this application are references to sources of electromagnetic (EM) radiation within the infrared range of the EM spectrum. As such, the term "light" is understood in its broad physical sense and is not limited to EM radiation visible to the human eye. Rather, the light sources in this context may be considered to be infrared light sources. The present invention has been developed based on research which has demonstrated that, as an alternative to electrical stimulation, nerve cells may be successfully stimulated by infrared (IR) radiation. In other words, instead of applying an electric current, the nerve cells may be stimulated to evoke a depolarization or an action potential by specific application of infrared radiation. As the application of IR radiation does not require an electrode, the present invention is thus able to eliminate the need for electrodes in a retinal implant entirely and thereby overcome the associated disadvantages described above. Although the precise mechanism by which the IR radiation stimulates the nerve cells is not yet fully understood, it is truly fascinating that retina nerve cells no longer functioning normally in visible light (i.e. that have lost their optical potential in visible light) may nevertheless be optically stimulated by light in the infrared spectrum.

In a preferred form of the invention, each of the light sources is configured to emit radiation in the near-infrared or mid-infrared range. In this connection, each of the light sources is desirably configured to emit radiation having a wavelength in the range of about 0.70 µm to 4.0 µm, more preferably in the range of about 0.75 µm to 3.0 µm and particularly preferably in the range of about 1.5 µm to 2.5 µm. The experimental results have indicated that IR radiation having a wavelength in the range of 1.8 µm to 2.2 µm is especially suitable for stimulation of ganglion cells in the retina. Varying the wavelength of the IR radiation has been found to have a significant impact on the penetration depth of the radiation, and this in turn can affect the amplitude of the action potentials generated depending on the depth in the tissue of the nerves to be stimulated. Thus, depending on the particular tissue of the subject to be treated, some degree of tuning of the stimulation wavelength may be required to produce optimal stimulation results. Desirably, the IR radiation is tuned or selected to penetrate the tissue to a depth in the range of 100 µm to 1 mm, and more preferably in the range of 200 µm to 600 µm. The larger wavelengths have been found to be less desirable because they can lead to excessive absorption of the IR radiation by water in the tissue, which reduces the penetration depth and general efficiency, as well as creating the potential for local temperature increases, which could be damaging.

Each of the plurality of light sources is adapted to emit the infrared radiation in pulses. The duration of the pulses is preferably in the range of 10 µs to 1 ms. Interestingly, shorter pulse durations have been found to require lower stimulation levels to evoke a given action potential. Furthermore, short pulse durations have the advantage of low radiant exposure, which can be particularly important for ensuring that the cells and tissue being stimulated does not experience any adverse thermal effects, i.e. caused by heating. For the purpose of artificially generating vision in a subject, each of the infrared light sources is preferably activated at a frequency of at least 1 Hz, more preferably at least 10 Hz, further preferably at least 25 Hz, and even more preferably at least 50 Hz. Because the pulse durations are relatively brief in comparison to the operating frequency of the light sources, each light source will be inactive, i.e. in an "off"-phase or not emitting, for the majority of the time. For example, a light source that operates at 50 Hz and emits pulses having a duration of 2 ms will be in an "off"-phase or not emitting for about 90% of the time.

In a preferred form of the invention, each of the plurality of light sources comprises a semiconductor laser, and more preferably a laser diode, e.g. a surface-emitting laser diode, such as a vertical-cavity surface-emitting laser (VCSEL) diode. VCSEL diodes are particularly suitable for use in a retinal implant of the invention because they can be fabricated with very small dimensions and can be readily integrated into a compact 2-D (i.e. 2-dimensional) array on a microchip, and because they emit light perpendicular to a plane or surface of the microchip. VCSEL diodes also have proven suitability for generating infrared radiation having a wavelength in the range of 1.3 µm to 2.0 µm. Further, laser diodes can generate a beam of infrared light having a small spot-size (e.g. with a diameter of about 100 µm or less, even 30 µm), thus providing for very specific stimulation of the nerve cells. In this regard, because the laser beams only stimulate the cells that they directly irradiate, they do also not generate any of the "cross-talk" typical with electrodes in the prior art. This enables the lasers to stimulate the nerve tissue much more specifically and to be arranged in a much more closely spaced array compared to electrodes, thereby providing the potential for higher resolution.

By combining the plurality of infrared light sources (e.g. laser diodes) in a microchip or integrated circuit, they may be pre-arranged in a 2D array on the chip to emit infrared radiation from a surface of the substrate to be directed towards the nerve cells of the retina. Each of the light sources can be controlled for independent actuation based on respective stimulation signals transmitted to or generated by the implant That is, the implant may be configured to receive respective stimulation signals transmitted to it, e.g. by optical or telemetric means, or to generate such stimulation signals itself, e.g. based on the image signal from the image processing means.

In a preferred form of the invention, the substrate comprises a web or film which carries the IR light sources and is configured to be implanted epiretinally. In this connection, the substrate may be formed so flexible that it readily adopts the curvature of the epiretinal surface when it is applied to the retina. Alternatively, the substrate may be pre-formed having a curvature adapted to the curvature of the epiretinal surface, e.g. using techniques disclosed in the co-pending International Patent Application No. PCT/EP2008/008225; International Publication Number WO 2010/034331). Thus, in a preferred form of the invention, the substrate may comprise at least two layers of material including a first layer and a second layer. The first and second layers in the substrate preferably consist of polymer material, such that the substrate of the implant or stimulation device preferably comprises a layered polymer web or film. Further, the first layer of material and the second layer of material may be selected to have different coefficients of thermal expansion to generate a desired curvature in the substrate.

Furthermore, there may be provided a method of manufacturing an implant or stimulation device according to the invention described above, including the steps of:
providing a first layer of material having a first coefficient of thermal expansion;
providing a second layer of material having a second coefficient of thermal expansion preferably different from the first coefficient of thermal expansion;
combining the first layer and the second layer to form a substrate, preferably at a temperature different to a normal service temperature or operation temperature of the implant or stimulation device; and
arranging a plurality of infrared light sources in an array on the substrate. The infrared light sources are preferably arranged to emit infrared radiation in a direction substantially perpendicular to a primary surface or plane of the substrate.

In a preferred form, the step of providing the first layer includes the step of applying the first layer of material on a base or support structure. Further, the step of providing the second layer includes the step of applying the second layer of material to the first layer.

Preferably, the step of arranging the plurality of IR light sources in an array on the substrate comprises placing and/or arranging the plurality of light sources individually. In an alternative preferred form, however, the step of arranging the plurality of IR light sources in an array on the substrate comprises: combining a prefabricated microchip or integrated circuit, on which the infrared light sources (e.g. laser diodes) have already been fixed in an array, with one or more layers of the substrate. The spacing between the individual laser diodes arranged in the array on the substrate is preferably less than or equal to about 500 µm, more preferably in the range of 100 µm to 400 µm, and even more preferably in the range of 200 µm to 300 µm. The microchip or integrated circuit may itself be provided as a flexible foil or wafer. The prefabricated microchip or integrated circuit may be sandwiched between the first layer and the second layer of the substrate. Alternatively, the microchip or integrated circuit may be applied and secured to an outer surface of a layer of the substrate. In either case, but particularly the latter, a sealing layer or coating may be applied over the microchip or integrated circuit to hermetically seal it from an aqueous environment within the body. Parylene is especially suitable for coating the laser diodes because it is substantially transparent to infrared light.

In a preferred form of the invention, the material(s) employed in the first and second layers of the substrate is/are polymer material(s), and more particularly, biocompatible polymer material(s). In this connection, the polymer material(s) is/are preferably selected from the group consisting of polyimide, parylene, and silicone. It will be appreciated that a polymer material selected for the substrate layers may be coated to ensure its biocompatibility. For example, a parylene coating may be applied to an outer surface of the substrate.

During production of the implant or stimulation device, the first and second layers of the substrate are preferably bonded, fused, cured or otherwise combined with one another in a flat condition at a temperature that is elevated compared to a normal operating temperature for the implant or stimulation device. Accordingly, a temperature differential exists (i.e. a change in temperature occurs) between that production phase and the normal operation of the implant. If the first and second material layers of the substrate have different coefficients of thermal expansion, this temperature change induces stresses or forces between the first and second layers of the substrate which act to deform or re-shape the substrate, and thereby endow the implant with a desired form. In particular, the substrate layer having the higher coefficient of thermal expansion will tend to form a concavely curved outer surface. In other words, because the materials of the first and second layers of the substrate are typically polymer materials which are bonded, fused and/or cured to form a layered structure at relatively high temperatures (e.g. in range of 200°C to 400°C) compared to room temperature (e.g. 22°C) or body temperature for a human or animal (e.g. 37°C) at which the implant typically operates, the temperature change between production and operation of the device will be a significant temperature reduction. Thus, the substrate layer having a higher coefficient of thermal expansion will tend to form a concavely curved outer surface. Where the stimulation device is intended to be employed as a retinal implant, in which the plurality of infrared light sources are to be directed outwardly from and/or through a second layer of the substrate having a convexly curved outer surface complementing a concave surface profile of the retina, the first layer of polymer material in the substrate will preferably have a higher coefficient of thermal expansion than the second layer.

The degree of curvature which is generated in the implant as a result of the different coefficients of thermal expansion of the first and second layers will depend, for example, upon the respective magnitude of the coefficient of thermal expansion (also called "CTE") of each of the first and second layers, as well as the thickness of each of these layers. The elasticity of the particular material(s) forming the layers will naturally also influence the degree of curvature generated. The CTE of the first layer may be in the range of about 20 ppm/°C (i.e. 20 x10⁻⁶/°C) to about 40 ppm/°C (i.e. 40 x10⁻⁶/°C). The CTE of the second layer, on the other hand, may be in the range of about 1 ppm/°C (i.e. 1 x10⁻⁶/°C) to 10 ppm/°C (i.e. 10 x10⁻⁶/°C), and more preferably in the range of 1 ppm/°C (i.e. 1 x10⁻⁶/°C) to 5 ppm/°C (i.e. 5 x10⁻⁶/°C). The elasticity of the first and second layers will typically be approximately equal.

In a preferred form of the invention, the first layer and the second layer extend with a substantially uniform thickness over the area of the substrate. Either of the first and second layers may itself have a layered structure and comprise multiple sub-layers. Preferably, the thickness of each layer and/or each sub-layer of the substrate is in the range of 1 µm to 100 µm, more preferably in the range of 1 µm to 50 µm, and particularly preferably in the range of 1 µm to 10 µm. The thickness of the first and second layers may be equal or may be adjusted as desired, but is usually within a ratio from about 1:1 to 1:5, or vice versa (i.e. 1:1 to 5:1).

When the retina stimulation device is implanted, the array of light sources (e.g. laser diodes) is preferably substantially centred in the region of the macula, where the retina has the greatest visual acuity and the greatest concentration of nerve cells. Preferably, fixation means are provided for fixing the substrate to the retina to hold the array of infrared light sources (e.g. laser diodes) in the desired position with respect to the macula. The fixation means may comprise biocompatible adhesive, or alternatively tacks, pins, staples or similar fastening elements. The fixation means are desirably applied spaced a distance away from the region of the retina to be stimulated so that any deleterious effect caused by the fixation means on the tissue does not affect the tissue and nerve cells to be stimulated.

As noted above, the substrate may include a semiconductor material and/or an integrated circuit or microchip for carry the plurality of light sources, e.g. laser diodes. The substrate preferably also comprises a sheath or coating of at least one polymer material for hermetically sealing sensitive components (e.g. electronics and circuitry) of the substrate from the aqueous environment within the eye. The polymer material may, for example, comprise one or more of a silicone, a parylene, and/or a polyimide. Furthermore, the sheath or coating may comprise multiple layers of such polymer material.

According to an alternative aspect, the present invention provides a medical implant device in the form of a pacemaker. Thus, the pacemaker of the invention comprises a substrate configured to be implanted in contact with heart tissue, and a plurality of infrared light sources arranged in an array on the substrate, wherein each of the light sources is configured to emit infrared radiation to stimulate muscle cells of the heart.

### Brief Description of the Drawings

The above and further features and advantages of the present invention will become more apparent from the following detailed description of particular embodiments of the invention with reference to the accompanying drawing figures, in which like components are designated with like reference characters, and in which:
- Figure 1: is a schematic plan view of an internal part of a visual prosthesis according to an embodiment of the present invention; and
- Figure 2: is a schematic side cross-sectional view of a retinal stimulation device in the embodiment of the visual prosthesis shown in Figure 1.

### Detailed Description of the Preferred Embodiments of the Invention

The visual prosthesis according to a preferred embodiment of the present invention incorporates both an "internal" part comprising components to be implanted in the body of the subject, and an "external" part comprising components to be carried or worn externally (i.e. non-implanted) by the subject. The basic system architecture of the visual prosthesis according to the invention generally reflects the state-of-the-art design described, for example, in International Patent Application Publication No. WO 2007/006376. As the details of the system architecture of the visual prosthesis are described in WO 2007/006376 at some length, much of that description will not be repeated here for the sake of economy. Rather, the reader should make direct reference to that document.

Thus, the visual prosthesis or system for artificially generating vision in a subject in this embodiment of the invention includes a device resembling a pair of glasses or spectacles (not shown) which incorporates image capture means in the form of a camera for capturing an image of the environment surrounding the user. The camera may, for example, incorporate a CCD or CMOS device, as is known in the art. The visual prosthesis further includes an external image processor (not shown) which may be incorporated in a small unit that is preferably designed to be carried by the subject, for example, in a breast pocket or in a belt-mounted pouch. The image processor device is operatively connected with the camera in the spectacles' frame and is designed to process and convert the images generated by the camera into image signals. The image signals are then transmitted telemetrically to the internal or implanted part of the visual prosthesis. That is, the frame of the spectacles may include a transmitter device for wirelessly transmitting the image signals to a signal processing device for converting the image signals into stimulation signals or stimulation impulses. The signal processing device is typically in the form of a micro-processor or micro-chip which may be implanted extra-ocularly in the user or subject, e.g. enclosed in a housing attached to an outer surface of the sclera.

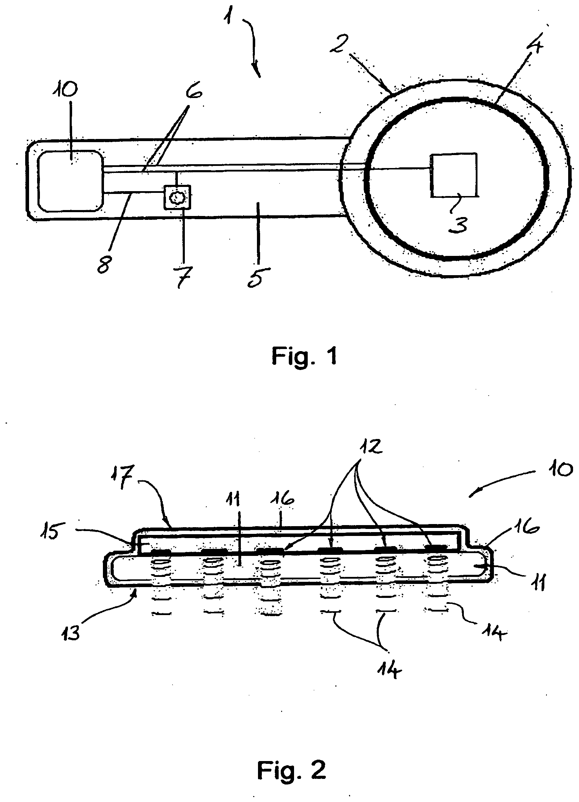

With reference now to Fig. 1 of the drawings, an internal part 1 of a visual prosthesis according to an embodiment of the invention is illustrated. The internal part 1 of the visual prosthesis shown in Fig. 1 includes a circular housing 2 which is configured to be implanted extra-ocularly in the subject, e.g. outside of, and possibly attached to, the sclera. The housing may be anchored in position using sutures or fastening bands, as it is known in the art. In this embodiment, the housing 2 includes a micro-processor or microchip 3 belonging to the signal processor device mentioned above for converting the image signals into stimulation signals or stimulation impulses. Further, a telemetry coil 4 is schematically shown in Fig. 1 overlying the housing 2. This coil 4 forms a receiver coil for receiving an RF or inductively transmitted data signal for the signal processor 3 and/or power signal for driving the internal part 1 of the visual prosthesis. In this regard, the housing 2 typically also incorporates circuitry for regulating the power supply for this internal part 1 of the prosthesis and a tuning capacitor for the receiver coil 4.

The housing 2 and the receiver coil 4 are physically and electrically connected to a retinal stimulation device 10 to be implanted adjacent the retina via an elongate flexible web 5. The elongate web 5 is formed of a polymer material incorporating electrical traces or wiring 6 so that it forms a kind of ribbon cable. As schematically illustrated in Fig. 1, the traces or wiring 6 provide electrical communication from the signal processor 3 and the coil 4 to the retinal implant or stimulation device 10, to be described in more detail later. Also provided on the ribbon cable is an infrared receiver-transmitter 7, which may comprise one or more photo-diodes, for receiving and/or transmitting data signals as described in WO 2007/006376. This receiver-transmitter 7 may be connected with the signal processor 3 via one of the traces 6 and/or may be separately connected with the retinal stimulation device 10, e.g. via another electrical trace or wiring 8.

Referring now to Fig. 2 of the drawings, the retinal stimulation device 10 according to a preferred embodiment of the invention is schematically illustrated in somewhat more detail. The stimulation device 10 comprises a substrate or support layer 11 formed from a flexible web of polymer material which is connected to, and may also be continuous with, the polymer web of the ribbon cable 5. Furthermore, the stimulation device includes a multitude of infrared light sources in the form of laser diodes 12, and in particular VCSEL diodes, which are arranged and fixed in a gridlike array (e.g. 50x50, 50x100, or 100x100) on the substrate 11. The plurality of laser diodes 12 are arranged with their laser-emitting surfaces facing substantially perpendicular to an outer surface 13 of the substrate 11. As the retinal stimulation device 10 is designed to be positioned with the outer surface 13 either in contact with, or directly adjacent to, the epiretinal surface, each of the infrared light sources (i.e. laser diodes 12) is configured to emit beams 14 of infrared (IR) radiation directly onto the tissue to be stimulated. The polymer material of the substrate 11 through which the beams 14 pass is therefore designed to be substantially transparent to infrared radiation, at least within the range of wavelengths emitted by the diodes 12, which in this case is in range from 1.8 µm to 2.2 µm.

The laser diodes 12 are themselves are preferably fixed on a microchip or integrated circuit 15 which is secured or bonded to the substrate 11. That microchip 15 is responsible for the switching, activation and control of the laser diodes 12 based on the stimulation signals derived from the image signal. Both the microchip 15 and the substrate 11 may be coated with a suitable polymer material to provide a hermetic sealing layer 16 which is also biocompatible. As this sealing layer 16 should also be transparent to IR radiation, parylene is especially preferred.

In operation, the image signal is transmitted telemetrically from the image processor in the external part of the visual prosthesis to the internal part 1 of the system, e.g. via RF transmission to the receiver coil 4 or via optical transmission to the receiver-transmitter 7. After that image signal has been transformed into stimulation signals or impulses in the signal processor microchip 3, those stimulation signals are then conveyed from the signal processor 3 to the control microchip 15 incorporating the array of laser diodes 12. Each of the diodes 12 is then individually or independently actuated to generate a beam 14 of IR radiation in dependence upon the stimulation signals to artificially generate a visual sensation for the subject corresponding to the image captured by the camera.

A possible modification or alternative embodiment of the system of this invention includes combining the circuitry for the power supply and signal processor 3 with the circuitry for controlling the infrared light sources, i.e. laser diodes 12. This could be achieved, for example, by incorporating all of this electronic control circuitry on a single microchip, namely the microchip 15 of the retina stimulation device. With sufficient miniaturization, it may then be possible to completely avoid the need for any extra-ocularly implanted housing 2 outside of the sclera.

## Claims

1. Retinal prosthesis comprising an internal part (1) configured to be implanted within an eye of a subject and positioned on or adjacent the retina, wherein the internal part (1) comprises a substrate (11),
**characterized in that** the internal part (1) further comprises a plurality of light sources (12) arranged in an array on the substrate (11),
wherein each of the light sources (12) is configured to emit infrared radiation in pulses with a duration in the range of 1 µs to 10 ms to optically stimulate nerve cells of the retina.

2. Retinal prosthesis according to claim 1, wherein each of the light sources (12) is configured to emit infrared radiation having a wavelength in the range of about 0.75 µm to 3.0 µm, and preferably in the range of 1.5 µm to 2.5 µm; and/or
wherein the plurality of light sources (12) are configured to be controlled for independent actuation based on stimulation signals transmitted to or generated by the prosthesis.

3. Retinal prosthesis according to claim 2, wherein the prosthesis is configured to receive telemetrically transmitted stimulation signals.

4. Retinal prosthesis according to any one of the preceding claims, wherein each of the plurality of light sources (12) comprises a laser diode, preferably a vertical-cavity surface-emitting laser (VCSEL) diode, and wherein the laser diodes are arranged in an array on a semiconductor material, such as an integrated circuit or microchip (15).

5. Retinal prosthesis according to any one of the preceding claims, wherein the substrate (11) comprises a flexible web or film, such as a polymer film, configured to be implanted epiretinally.

6. Retinal prosthesis according to any one of the preceding claims, wherein the substrate (11) comprises at least one layer or coating (16) of a material that hermetically seals the plurality of light sources (12) from an aqueous environment within the eye.

7. Retinal prosthesis according to any one of the preceding claims, wherein the substrate (11) is formed having a curvature that is adapted to a curvature of the retina, wherein the curvature of the substrate is preferably a result of a temperature influence and/or a coefficient of thermal expansion of the substrate material.

8. Retinal prosthesis according to any one of the preceding claims, wherein fixation means are provided for fixing the substrate (11) to the retina, the fixation means preferably comprising tacks or similar fastening elements.

9. System for generating artificial vision in a subject, comprising:
image capture means for capturing an image from a surrounding environment;
image processing means for processing the image and converting the image into an image signal; and
a retinal prosthesis according to any one of the preceding claims configured to be implanted within an eye of a patient and positioned on or adjacent the retina, the prosthesis comprising a substrate (11)
**characterized in that** the prosthesis further comprises a plurality of light sources (12) arranged in an array on the substrate (11) for stimulating nerve cells of the retina, wherein each of the plurality of light sources (12) is configured to emit infrared radiation to stimulate one or more nerve cells in response to a respective stimulation signal derived from the image signal.

10. System according to claim 9, further comprising signal processing means (3) for converting the image signal into a plurality of stimulation signals for activating the plurality of light sources.

11. System according to claim 10, wherein the system comprises an external part which is to be worn or carried externally on the body of the subject, and an internal part which is to be implanted in the body of the subject, wherein the image capture means and the image processing means form components of the external part, and wherein the retinal prosthesis and the signal processing means (3) are components of the internal part.

12. System according to claim 11, wherein the external part further comprises signal transmission means for transmitting the image signal to the image processing means, the signal transmission means including an RF or optical telemetry means.

13. System according to any one of claims 9 to 12, wherein each of the plurality of light sources (11) comprises a laser diode, preferably a vertical-cavity surface-emitting laser (VCSEL) diode, wherein the laser diodes are integrated on a microchip and arranged to emit IR radiation substantially perpendicular to a surface of the substrate.

## Patentansprüche

1. Retinaprothese, umfassend einen internen Teil (1), der eingerichtet ist, in das Auge eines Subjekts implantiert und auf der oder angrenzend an die Retina positioniert zu werden, wobei der interne Teil (1) ein Substrat (11) umfasst,
**dadurch gekennzeichnet, dass** der interne Teil (1) des Weiteren eine Mehrzahl an Lichtquellen (12) umfasst, die in einem Array auf dem Substrat (11) angeordnet sind,
wobei jede der Lichtquellen (12) eingerichtet ist, um Infrarotstrahlung in Impulsen mit einer Dauer im Bereich von 1 µs bis 10 ms zu emittieren, um Nervenzellen der Retina optisch zu stimulieren.

2. Retinaprothese nach Anspruch 1, wobei jede der Lichtquellen (12) eingerichtet ist, Infrarotstrahlung zu emittieren, die eine Wellenlänge im Bereich von etwa 0,75 µm bis 3,0 µm und vorzugsweise im Bereich von 1,5 µm bis 2,5 µm aufweist; und/oder
wobei die Mehrzahl der Lichtquellen (12) für eine unabhängige Betätigung, basierend auf Stimulationssignalen, die zu der Prothese übertragen oder von dieser erzeugt werden, kontrollierbar eingerichtet ist.

3. Retinaprothese nach Anspruch 2, wobei die Prothese eingerichtet ist, telemetrisch übermittelte Stimulationssignale zu empfangen.

4. Retinaprothese nach einem der vorhergehenden Ansprüche, wobei jede der Mehrzahl der Lichtquellen (12) eine Laserdiode, vorzugsweise eine Vertikalkavitätsoberflächenemittierende Laserdiode (VCSEL-Diode) umfasst, und wobei die Laserdioden in einem Array auf einem Halbleitermaterial, wie z.B. einem integrierten Schaltkreis oder einem Mikrochip (15), angeordnet sind.

5. Retinaprothese nach einem der vorhergehenden Ansprüche, wobei das Substrat (11) ein/en epiretinal implantierbar eingerichtetes/n flexibles/n Netz ("web") oder Film, wie z.B. einen Polymerfilm, umfasst.

6. Retinaprothese gemäß einem der vorhergehenden Ansprüche, wobei das Substrat (11) wenigstens eine Schicht oder Beschichtung (16) eines Materials umfasst, das die Mehrzahl an Lichtquellen (12) hermetisch von einer wässrigen Umgebung innerhalb des Auges abdichtet.

7. Retinaprothese gemäß einem der vorhergehenden Ansprüche, wobei das Substrat (11) mit einer Krümmung geformt ist, die an eine Krümmung der Retina angepasst ist, wobei die Krümmung des Substrats vorzugsweise ein Resultat eines Temperatureinflusses und/oder eines thermischen Expansionskoeffizienten des Substratmaterials ist.

8. Retinaprothese gemäß einem der vorhergehenden Ansprüche, wobei Fixiermittel zum Fixieren des Substrats (11) an der Retina vorgesehen sind, wobei die Fixiermittel vorzugsweise Nägel oder ähnliche Befestigungselemente umfassen.

9. System zur Erzeugung von künstlichem Sehvermögen in einem Subjekt, umfassend:
Bildaufnahmemittel zum Aufnehmen eines Bildes von einer umgebenden Umwelt;
Bildbearbeitungsmittel zum Bearbeiten des Bildes und Konvertieren des Bildes in ein Bildsignal; und
eine Retinaprothese gemäß einem der vorhergehenden Ansprüche, die eingerichtet ist, in ein Auge des Patienten implantiert und auf der oder angrenzend an die Retina positioniert zu werden, wobei die Prothese ein Substrat (11) umfasst,
**dadurch gekennzeichnet, dass** die Prothese des Weiteren eine Mehrzahl an Lichtquellen (12) umfasst, die zur Stimulation von Nervenzellen der Retina in einem Array auf dem Substrat (11) angeordnet sind, wobei jede der Mehrzahl an Lichtquellen (12) eingerichtet ist, Infrarotstrahlung zu emittieren, um eine oder mehrere Nervenzellen als Reaktion auf ein entsprechendes Stimulationssignal, das von dem Bildsignal abgeleitet wird, zu stimulieren.

10. System nach Anspruch 9, des Weiteren umfassend Signalverarbeitungsmittel (3) zum Konvertieren des Bildsignals in eine Mehrzahl an Stimulationssignalen zur Aktivierung der Mehrzahl an Lichtquellen.

11. System gemäß Anspruch 10, wobei das System einen externen Teil, der extern an dem Körper des Subjekts anzulegen oder zu tragen ist, und einen internen Teil umfasst, der in den Körper des Subjekts implantierbar ist, wobei die Bildaufnahmemittel und die Bildbearbeitungsmittel Komponenten des externen Teils bilden, und wobei die Retinaprothese und die Signalverarbeitungsmittel (3) Komponenten des internen Teils sind.

12. System gemäß Anspruch 11, wobei der externe Teil des Weiteren Signalübertragungsmittel zum Übertragen des Bildsignals zu den Bildbearbeitungsmitteln umfasst, wobei die Signalübertragungsmittel RF (Hochfrequenz)- oder optisches Telemetriemittel enthalten.

13. System gemäß einem der Ansprüche 9 bis 12, wobei jede der Mehrzahl an Lichtquellen (11) eine Laserdiode, vorzugsweise eine Vertikalkavitätsoberflächenemittierende Laserdiode (VCSEL-Diode) umfasst, wobei die Laserdioden auf einem Mikrochip integriert sind und angeordnet sind, IR-Strahlung im Wesentlichen senkrecht zu einer Oberfläche des Substrats zu emittieren.

## Revendications

1. Prothèse rétinienne comprenant une partie interne (1) configurée pour être implantée dans un oeil d'un sujet et positionnée sur la rétine ou adjacente à celle-ci, dans laquelle la partie interne (1) comprend un substrat (11),
**caractérisée en ce que** la partie interne (1) comprend en outre une pluralité de sources de lumière (12) agencées en un réseau sur le substrat (11),
dans laquelle chacune des sources de lumière (12) est configurée pour émettre des impulsions de rayonnement infrarouge ayant une durée de 1 µs à 10 ms pour stimuler optiquement les cellules nerveuses de la rétine.

2. Prothèse rétinienne selon la revendication 1, dans laquelle chacune des sources de lumière (12) est configurée pour émettre un rayonnement infrarouge ayant une longueur d'onde dans la plage d'environ 0,75 µm à 3,0 µm, et de préférence dans la plage de 1,5 µm à 2,5 µm ; et/ou
dans laquelle la pluralité de sources de lumière (12) sont configurées pour être commandées pour une activation indépendante sur la base de signaux de stimulation transmis à la prothèse ou générés par celle-ci.

3. Prothèse rétinienne selon la revendication 2, dans laquelle la prothèse est configurée pour recevoir des signaux de stimulation transmis de manière télémétrique.

4. Prothèse rétinienne selon l'une quelconque des revendications précédentes, dans laquelle chacune de la pluralité de sources de lumière (12) comprend une diode laser, de préférence une diode laser à émission de surface à cavité verticale (VCSEL), et dans laquelle les diodes laser sont agencées en un réseau sur un matériau semi-conducteur, tel qu'un circuit intégré ou une micropuce (15).

5. Prothèse rétinienne selon l'une quelconque des revendications précédentes, dans laquelle le substrat (11) comprend une bande ou un film souple, tel qu'un film de polymère, configuré pour être implanté de manière épirétinale.

6. Prothèse rétinienne selon l'une quelconque des revendications précédentes, dans laquelle le substrat (11) comprend au moins une couche ou un revêtement (16) d'un matériau qui enferme hermétiquement la pluralité de sources de lumière (12) par rapport à un environnement aqueux dans l'oeil.

7. Prothèse rétinienne selon l'une quelconque des revendications précédentes, dans laquelle le substrat (11) est formé de manière à présenter une courbure qui est adaptée à une courbure de la rétine, dans laquelle la courbure du substrat est de préférence un résultat d'une influence de la température et/ou d'un coefficient de dilatation thermique du matériau de substrat.

8. Prothèse rétinienne selon l'une quelconque des revendications précédentes, dans laquelle des moyens de fixation sont prévus pour fixer le substrat (11) à la rétine, les moyens de fixation comprenant de préférence des clous ou des éléments de fixation similaires.

9. Système pour générer une vision artificielle dans un sujet, comprenant :
des moyens de capture d'image pour capturer une image d'un environnement alentour ;
des moyens de traitement d'image pour traiter l'image et convertir l'image en un signal d'image ; et
une prothèse rétinienne selon l'une quelconque des revendications précédentes configurée pour être implantée dans un oeil d'un patient et positionnée sur la rétine ou adjacente à celle-ci, la prothèse comprenant un substrat (11),
**caractérisé en ce que** la prothèse comprend en outre une pluralité de sources de lumière (12) agencées en un réseau sur le substrat (11) pour stimuler les cellules nerveuses de la rétine, dans lequel chacune de la pluralité de sources de lumière (12) est configurée pour émettre un rayonnement infrarouge pour stimuler une ou plusieurs cellules nerveuses en réponse à un signal de stimulation respectif déduit du signal d'image.

10. Système selon la revendication 9, comprenant en outre des moyens de traitement de signal (3) pour convertir le signal d'image en une pluralité de signaux de stimulation pour activer la pluralité de sources de lumière.

11. Système selon la revendication 10, dans lequel le système comprend une partie externe qui doit être portée ou supportée extérieurement sur le corps du sujet, et une partie interne qui doit être implantée dans le corps du sujet, dans lequel les moyens de capture d'image et les moyens de traitement d'image forment des composants de la partie externe, et dans lequel la prothèse rétinienne et les moyens de traitement de signal (3) sont des composants de la partie interne.

12. Système selon la revendication 11, dans lequel la partie externe comprend en outre des moyens de transmission de signal pour transmettre le signal d'image aux moyens de traitement d'image, les moyens de transmission de signal comprenant des moyens de télémétrie RF ou optique.

13. Système selon l'une quelconque des revendications 9 à 12, dans lequel chacune de la pluralité de sources de lumière (11) comprend une diode laser, de préférence une diode laser à émission de surface à cavité verticale (VCSEL), dans lequel les diodes laser sont intégrées sur une micropuce et agencées pour émettre un rayonnement IR sensiblement perpendiculaire à une surface du substrat.
